# EUROPEAN PATENT APPLICATION

(11) **EP 2 842 932 A1**
(43) Date of publication of application: **04.03.2015**
(21) Application number: 12875656.6
(22) Date of filing: 27.04.2012
(51) Int. Cl.: C07C 67/03, C07C 69/68

(54) **METHOD FOR CONTINUOUSLY PRODUCING HIGH-CONTENT HIGH-OPTICAL-PURITY LACTATE**

(71) Applicant: Xiaogan Esun New Material Co., Ltd., Xiaogan, Hubei 432001 (CN)
(72) Inventor: YANG, Yihu, Xiaogan Hubei 432001 (CN); XU, Jie, Xiaogan Hubei 432001 (CN)
(74) Representative: Laufhütte, Dieter
(86) International application number: PCT/CN2012/074847
(87) International publication number: WO 2013/159347

(57) **Abstract**

A continuous method for directly producing lactate through an ester exchange reaction. The method comprises the steps of: adding catalyst in lactic acid to allow for polycondensation so as to obtain lactic acid oligomer having a molecular weight of 1000-2500; adding catalyst into the lactic acid oligomer to allow fordepolymerization so as to obtain crude lactide; and refining the crude lactide, and subjecting the refined lactide and organic alcohol to full-reflux reaction to obtainlactate. The method of the present invention is simple in process, easy to perform, and suitable for industrial production and application. The materials can all be utilized. No "three wastes" emission occurs. Content of the synthesized lactate of the present invention is more than 99.5%, and optical purity of the lactate can reach more than 99%.

## Description

### FIELD OF THE INVENTION

The invention belongs to the technical field of organic chemical industry, and more particularly to a method for directly and continuously producing a high-content high-optical-purity lactate by transesterfication, with refined lactide and anhydrous alcohol as raw materials.

### BACKGROUND OF THE INVENTION

Lactate has a special rum, fruit, and cream flavor, and is an important fine chemical raw material. Since the lactate contains asymmetric carbon atoms, it has two optical isomers. Lactate is an important industrial solvent possessing optical activity and can be used as a solvent for nitrocellulose, cellulose acetate, alkyd resin, kauri resin, Manila resins, rosin, shellac, vinyl resins, or paints, as well as a senior solvent for artificial pearl.Lactate is also utilized as a plasticizer in other industrial field. It can be used to synthesize carboxylic acid ester having the optical activity in asymmetrical synthesis, or used as lubricants for pressing pills or as intermediates for producing drugs in pharmacy. Some Lactates of high alcohol is often applied in cosmetics due to its small irritation, less volatile, good spreadability, good water solubility, and compatibility with other water soluble content.Thus, the lactate is widely used in food, wine, chemical, pharmaceutical and other industries.

Meanwhile,characterized in its non-poisonous, good solubility, less volatile, fruit flavor, and biodegradability, the lactate is also a green solvent having great development value and application prospect. With the continuous development of people's living standard, the environment is more and more highly required. However, most industrially used solvents are poisonous solvents that seriously endangerthe environment and human beings, for example, halogenated hydrocarbons, ethers, and chlorofluorocarbon solvents.Thus, the lactate has a broad application prospect when it is used as the green solvent, and the electronic grade ethyl lactate has been widely applied in the electronics cleaning industry.

Lactate synthesis technology has been reported.Chinese Patent Publication No. CN1229790Ahas disclosed a method for synthesizing ethyl lactate using nature raw materials.Chinese Patent Publication No. CN1720215A has disclosed a method for continuously preparing ethyl lactate. Chinese Patent Publication No. CN1438213A has disclosed a process for preparing ethyl lactate using ammonium lactate as a raw material.Chinese Patent Publication No. CN1102180A has disclosed a new process for producing ethyl lactate.Chinese Patent Publication No. CN1114035 has disclosed a process for preparing ethyl lactate by distillation.Chinese Patent Publication No.CN1290686 has disclosed a method for synthesizing ethyl lactate catalyzed by ammonium lactate.Chinese Patent Publication No. CN1594585 has disclosed a method for enzymetic synthesizing ethyl lactate in a solution phase. Chinese Patent Publication No. CN1613842 has disclosed a new process for enzymetic synthesizing ethyl lactate by distillation.Chinese Patent Publication No.CN1740331has disclosed a method for producing ethyl lactate and joint product thereof by biorefinery.Chinese Patent Publication No. CN101575622 has disclosed a process for producing ethyl lactate from ammonium lactate. Chinese Patent Publication No. CN1290686A has disclosed a method for synthesizing ethyl lactate catalyzing by ammonium lactate. Chinese Patent Publication No. CN101759559A has disclosed a method for preparing n-propyl propionate having low water content, low acidity, and high content.Chinese Patent Publication No. CN1450046 has closed a method for synthesizing L-butyl lactate having high yield and high optical purity.

However, the prior arts disclose neithera method for producing high optical purified ethyl lactateby transesterificationusing lactide and anhydrous ethanol as the raw material, nor a process for producing electronic grade ethyl lactate using a mother liquid of lactide and anhydrous ethanol.

### SUMMARY OF THE INVENTION

In view of the above-described problems, it is one objective of the invention to providea method for continuously producing a high-content high-optical-purity lactate. It is another objective of the invention to provide a method for producing electronic grade ethyl lactate.The method of the invention has simple process and operation, and is adaptable to industrial production and application. Raw materials are completely utilized, and no waste water, waste gas, or waste residue is discharged.The invention firstly adopts lactic acid as the raw material to synthesize lactide, and uses a refined lactide and anhydrous ethanol as the raw material to synthesize the lactate.A purified mother liquid of the lactate and anhydrous ethanol are used as the raw material to synthesize ethyl lactate.The whole process routing can be divided into a production process of lactide and a production process of the lactate.

Technical scheme of the invention is as follows: a method for continuously producing a high-content high-optical-purity lactate, comprises the following steps:

A. polycondensation of lactic acid: adding a first catalyst to lactic acid, controlling a weight ratio of the first catalyst to lactic acid to be between 0.1/10000 and 10/10000, and conducting a polycondensation reaction, whereby yielding a lactic acid oligomer having amolecular weight of between 1000 and 2500;

B. depolymerization of an oligomer: adding a second catalyst to the lactic acid oligomer, controlling a weight ratio of the second catalyst to the lactic acid oligomer to be between 0.1/10000 and 10/10000, and conducting a depolymerization reaction, whereby yielding a crude lactide having a content of between 80 and 95 wt. %;

C. purification of lactide: refining the crude lactide to yield a refined lactide having a content of 99.5 wt. % above and an optical purity of 99.5 wt. % above and a purified mother liquid of an acyl lactate;

D. synthesis of a high-optical-purity lactate: adding the refined lactide and an anhydrous organic alcohol at a weight ratio of between 1:0.7 and 1:5, preferably 1:3, to a dissolution kettle to completely dissolve lactide into the organic alcohol; introducing a resulting alcohol solution comprising lactide to pass through a fixed bed reactor added with a third catalyst so thattransesterification reaction between the lactide and the organic alcohol occurs, whereby yielding a lactate;

E. synthesis of electronic grade ethyl lactate: evenly mixing the purified mother liquid of the acyl lactate and anhydrous ethanol at a weight ratio of between 1:0.7 and 1:5 to yield a mixture, introducing the mixture to pass through a fixed bed reactor added with a fourth catalyst to obtain ethyl lactate; and

F. purification of ethyl lactate: conducting purification and separation of ethyl lactate.

As an improvement of the invention, vacuum distillation is conducted on lactic acid at a vacuum degree of between 10000 and 500 Pa and a distillation temperature of between 40 and 90°C before the polycondensation of lactic acid, and free water is removed therefrom.

The first catalyst added in the polycondensation of lactic acid and the second catalyst added in the depolymerization of the oligomer are selected from the group consisting of a zinc catalyst, a tin catalyst, a titanium catalyst, an organic acid catalyst, and a mixture thereof. A weight ratio of the first catalyst to the lactic acid is between 0.1/1000 and 50/10000. A weight ratio of the second catalyst to the lactic acid oligomer is between 0.1/1000 and 50/10000.Preferably, the first catalyst added in the polycondensation of lactic acid and the second catalyst added in the depolymerization of the oligomer are selected from the group consisting of zinc lactate, zinc oxide, zinc dust, diethyl zinc, tin lactate, tin oxide, tin dioxide, stannous oxide, stannous lactate, stannous octoate, stannous chloride, tin powder, propionic acid, butyl titanate, and a composite catalyst thereof.Preferably, the catalyst is a mixture of zinc oxide, tin oxide, and butyl titanate at a weight ratio of 1:1:1.Preferably, the catalyst is a mixture of zinc lactate, propionic acid, and butyl titanate at a weight ratio of 1:1:1.

The third catalyst added in the fixed bed reactor in the synthesis of the lactate is selected from the group consisting of a hydrogen-type cation exchange resin, a SO₄²⁻/TI₂O₄ solid high acid catalyst, and a composite catalyst thereof.Preferably, the composite catalyst comprises the hydrogen-type cation exchange resin and the SO₄²⁻/TI₂O₄ solid high acid at a weight ratio of 1:2.

Therefining of the crude lactide in step C) adopts recrystallization, vacuum distillation, or melt crystallization, or a combination thereof.Preferably, vacuum distillation followed by melt crystallization is adopted.

The fixed bed reactor for the synthesis of the lactate in step D) or E) is a tubular fixed bed reactor. The fixed bed reactor is a single-stage fixed bed reactor, or a multi-stage fixed bed reactor formed by a series connection of two or more tubular fixed bed reactors.

Purification and separation of the lactate in step F) is performed using a tri-column distillation process comprising: removing excessive alcohol in a first distillation column, removing the acyl lactate in a second distillation column, and removing remaining alcohol in a third distillation column. The first distillation column operates in decompression at a vacuum degree of between -0.02 and -0.08mPa, and an alcohol content accounts for between 1 and 5 wt. % of materials in a bottom of the first distillation column.The second distillation column operates in decompression at a vacuum degree of between -0.086 and -0.098mPa, and a lactate content accounts for between 5 and 20 wt. % of materials in a bottom of the second distillation column.The third distillation column operates at normal pressure, an alcohol content accounts for 0.1 wt. % of products in a bottom of the third distillation column. The lactate content reaches 99.8 wt. % after the tri-column distillation process.

The raw material lactic acid is L-lactic acid or D-lactic acid.If L-lactic acid is used as the raw material, a refined L-lactide is obtained, and a final product is L-lactate.If D-lactic acid is used as the raw material, a refined D-lactide is obtained, and a final product is D-lactate.

The anhydrous organic alcohol is anhydrous organic alcohol having reactivity, such as methanol, ethanol, propanol, butanol, pentanol, hexanol, heptanol, octanol, nonanol, decanol, undecyl alcohol, dodecyl alcohol, tridecyl alcohol, hexadecanol, stearyl alcohol, lauryl alcohol, menthol, and glycerol alcohol.

Compared with the prior art, advantages of the invention are summarized as follows:

1. The process for producing high-optical purity of the invention uses the refined lactide and anhydrous alcohol as the direct raw material, which is different from the conventional process that uses lactic acid and alcohol as the direct raw material. Compared with the conventional process, the process of the invention is characterized in that:First, The intermediate lactide is produced using lactic acid as the raw material.It is known that lactic acid has two isomers, L-lactic acid and D-lactic acid have almost the same physical and chemical properties, and it is difficult to separate one from the other by using the common separation processes. But the optical content of lactic acid directly affects the optical content of the product methyl lactate.Lactide has two isomers and exists in an L-form, a D-form, a racemic-form, and a meso-form. L-lactide and D-lactide have fusion points of between 95 and 98°C, racemic lactide has a fusion point of between 124 and 126°C, and meso lactide has a fusion point of between 50and 52°C. Solubilities of the four existing forms of lactide in the same solvent at the same temperature are different from one another; in which, the meso-form ranks the first, followed by the L-form and the D-form, and the racemic-form is the last.Also, the boiling points of the four existing forms of lactide also distinct from one another.Thus, the separation of different existing forms of lactide can be realized by the common separation means.The purification separation in the process of the invention utilizesrecrystallization of solvent, vacuum distillation, melt crystallization, or a combination thereof, and the content of the refined lactide reaches 99.5 wt. %, an optical purity thereof reaches 99.5% above, and a water content reaches 0.05 wt. %.Second, anhydrous alcohol and the refined lactide as the raw material of the transesterification reaction have very low water contents, and no water is produced in the transesterification reaction. Thus, water agent is not demanded in the whole reaction, the reaction carries out completely, and the water content in the product lactate is very low.Third, the refined lactide has the optical purity of 99.5% above, isomers conversion will not occur in the transesterification reaction.Thus, the optical purity of the synthesized lactate reaches 99.5% above.

2. Electronic grade ethyl lactate is produced by using the purified mother liquid of lactide and anhydrous ethanol, and is characterized in that: first, the purified mother liquid and anhydrous ethanoldo not contain water, and no water is produced in the transesterification reaction, so that water agent is not demanded in the whole reaction, and the water content in the product can be 200 ppm below. Second, lactide is used to produce the high purity lactate, and the purified mother liquid is used to produceethyl lactate, so that all the raw materials are effectively utilized.

3. The third catalyst added in the fixed bed reactor in the synthesis of the lactate is selected from the group consisting of the hydrogen-type cation exchange resin, the SO₄²⁻/TI₂O₄ solid high acid catalyst, and the composite catalyst thereof.Compared with concentrated sulfuric acid used in the conventional process, thethird catalyst of the invention is advantageous in that:First, high efficiency, the reaction rate can be increased by adding the catalyst accounting for only between 10/1000 and 100/1000 of the weight of lactide.Second, the catalyst can be reused, when the catalyst effect is decreased after a long term use, it only requires filtration separation, desiccation, and simple activation process to reuse the catalyst.Third, the catalyst is not corrosive to the devices, thereby largely decreasing the investment costs on the devices.Fourth, such catalystfunctions only in facilitating the reaction rather than enabling the reactants and the products to participate in other side reactions.The mother liquid of the lactate only contains non-reacted alcohol, product lactate, and the lactoyl lactate produced from incomplete reaction, so that the difficulty for distillation separation is largely decreased.Fifth, no waste acid is discharge, thereby being pollution free.

4. The fixed bed is used as the reactor for synthesizing lactate. The whole production can be continuously conducted, thereby increasing the energy production per unit, decreasing the difference between different batches, and ensuring stable product quality.

5. The separation purification of the lactate is performed using the tri-column distillation process.The former two distillation columns operate in decompression conditions, the production temperature is lowered, and the probability of the occurrence of side reactions in the raw materials comprising a plurality of impurities is decreased. The third distillation column adopts normal pressure distillation to further remove alcohol from the product.From the tri-column distillation, the content of the lactate reaches 99.8 wt. % above, and the alcohol content therein is decreased to 0.1 wt. % below.

6. The process routing of the invention is environmentally friendly and pollution free.First, the distilled water produced in the polycondensation of lactic acid completely satisfies the water demands for normal production and washing. The whole process routing does not need additional clean water.Second, the catalysts can be reused and no side reactions between the catalyst and the reactants occur, so that emission of the deactivated catalyst and the reaction byproducts does not occur.Third, the three components in the mother liquid after the distillation reaction are completely converted into product, the lactate is the final product, the recovered alcohol and the lactoyl lactate are returned to the transesterification section for reaction, so that the emission problems of the waste water, waste gas, and waste residue does not exist.

### DETAILED DESCRIPTION OF THE EMBODIMENTS

For further illustrating the invention, experiments detailing a method for continuously producing a high-content high-optical-purity lactate are described hereinbelow combined with the drawings.

### Example 1

88 wt. % of L-lactic acid was purchased and used as a raw material.The raw material lactic acid was performed with vacuum distillation in a falling film evaporator.The temperature of the raw material in the falling film evaporator was maintained at 50°C, and a vacuum degree was maintained at 8000 Pa. The lactic acid after condensation contained 99 wt. % above of its monomer.500 kg of the lactic acid after condensation was transported to a polycondensation kettle via a first delivery pump, and zinc lactate was added as a catalyst with a weight ratio of zinc lactate to the lactic acid of 5/10000.Materials were heated from 80°C to 120°C within 2 hours, and the vacuum degree was maintained at 5000 Pa.The whole reaction was performed in the stirring condition.The viscosity of a lactic acid oligomer was measured via an on line viscometer to determine whether a molecular weight thereof was between 1000 and 2500 according to corresponding relation between the viscosity and the molecular weight.If the molecular weight fell within the range, the lactic acid oligomer was transported to a depolymerization kettle by a height pressure difference and a vacuum pressure difference. Zinc lactate was added as the catalyst with a weight ratio of zinc lactate to the lactic acid of 5/10000.The whole reaction was conducted in the stirring condition.Raw materials were rapidly heated to the temperature of 180°C, and the vacuum degree was controlled at 1000 Pa.Lactide vapor was continuously obtained, condensed, and accumulated in a collection tank.The depolymerization reaction stopped when the materials in the depolymerization kettle were completely depolymerized and the formation of the lactide became very slow.A liquid lactide in the collection tank was transported to a purification section via a second delivery pump where the liquid lactide was conducted with recrystallization purification process, and 385 kg of a refined lactide having a content of 99.7 wt. % above and an optical purity of 99.7% was obtained.The 385 kg of the refined L-lactide was transported to a dissolution kettle via a third delivery device while 500 kg of anhydrous methanol was added to the dissolution kettle.Materials therein were heated to the temperature of 75°C. After thematerials were completely dissolved,a fourth delivery pump operated to introduce the methanol solution comprising lactide to pass through a fixed bed reactor added with a cation exchange resin for performing transesterification reaction.A mother liquid after the reaction was introduced to a buffer tank and was then continuously transported to distillation columns via afifthdelivery pump.The temperature of materials in a bottom of a first distillation column was controlled at between 140°C and 141°C, the temperature of a top of the first distillation column was controlled between 65 and 65.5°C, and a pressure difference between the top and the bottom thereof was controlled at 1.8 kPa.Methanol was recovered at the top of the first distillation column, and a product produced in the bottom of the first distillation column was transported to a second distillation column.The temperature of materials in the second distillation column was controlled at between 90 and 95°C, the temperature of the top in the second distillation column was controlled at between 65 and 70°C, and the vacuum degree therein was controlled at -0.09 mPa.Heavy components were continuously obtainedin the bottom of the second distillation column. Products produced in the top of the second distillation column were introduced into a third distillation column.The temperature in a bottom of the third distillation column was controlled at between 144 and 146°C, methanol was continuously recovered from a top of the third distillation column, and L-methyl lactate was continuously obtained from the bottom of the third distillation column.After the distillation, 600 kg of L-methyl lactate having a content of 99.85 wt. % and an optical purity of 99.9% was obtained.Lactoyl methyl lactate and recovered methanol were returned to the methyl lactate synthesis system to participate the synthesis of methyl lactate.

### Example 2

5000 kg of the lactic acid after condensation was transported to a polycondensation kettle via a first delivery pump, and zinc oxide was added as a catalyst with a weight ratio of zinc oxide to the lactic acid of 10/10000.Materials were heated from 80°C to 120°C within 2 hours, and the vacuum degree was maintained at 4000 Pa.The whole reaction was performed in the stirring condition.The viscosity of a lactic acid oligomer was measured via an on line viscometer, and a molecular weight thereof was determined to be 1600 according to corresponding relation between the viscosity and the molecular weight.After the polycondensation reaction, the lactic acid oligomer was transported to a depolymerization kettle by a height pressure difference and a vacuum pressure difference. Zinc oxide was added as the catalyst with a weight ratio of zinc oxide to the lactic acid of 10/10000.The whole reaction was conducted in the stirring condition.Raw materials were rapidly heated to the temperature of 185°C, and the vacuum degree was controlled at 1500 Pa.Lactide vapor was continuously obtained, condensed, and accumulated in a collection tank.The depolymerization reaction stopped when the materials in the depolymerization kettle were completely depolymerized and the formation of the lactide became very slow.A liquid lactide in the collection tank was transported to a purification section via a second delivery pump where the liquid lactide was conducted with melt crystallization purification process, and 3800 kg of a refined D-lactide having a content of 99.6 wt. % above and an optical purity of 99.6% was obtained.The 3800 kg of the refined D-Iactide was transported to a dissolution kettle via a third delivery device while 10000 kg of anhydrous isobutanol was added to the dissolution kettle.After thematerials were completely dissolved, a fourth delivery pump operated to introduce the isobutanol solution comprising lactide to pass through a fixed bed reactor added with a cation exchange resin for performing transesterification reaction.A mother liquid after the reaction was introduced to a buffer tank and was then continuously transported to distillation columns via a fifth delivery pump. The temperature of materials in a bottom of a first distillation column was controlled at between 140°C and 141°C, the temperature of a top of the first distillation column was controlled between 85 and 85.5°C, and the vacuum degree therein was controlled at -0.086 mPa.Isobutanol was recovered at the top of the first distillation column, and a product produced in the bottom of the first distillation column was transported to a second distillation column.The temperature of materials in the second distillation column was controlled at between 90 and 95°C, the temperature of the top in the second distillation column was controlled at between 65 and 70°C, and the vacuum degree therein was controlled at -0.095 mPa. Heavy components were continuously obtained in the bottom of the second distillation column.Products produced in the top of the second distillation column were introduced into a third distillation column.The temperature in a bottom of the third distillation column was controlled at between 160 and 162°C, isobutanol was continuously recovered from a top of the third distillation column, and D-isobutyl lactate was continuously obtained from the bottom of the third distillation column.After the distillation, 7000 kg of D-isobutyl lactate having a content of 99.8wt. % and an optical purity of 99.9% was obtained.Lactoyl isobutyl lactate and recovered isobutyl lactate were returned to the isobutyl lactate synthesis system to participate the synthesis of isobutyl lactate.

### Example 3

A recovered mother liquid of lactide andanhydrous ethanol at a weight ratio of 1:1.6 were added to a dissolution kettle and were evenly mixed to yield a mixed solution. A fourth delivery pump operated to transport the mixed solution to pass through a fixed bed reactor added with a SO₄²⁻/TI₂O₄ solid high acid catalyst at a flow rate of 600 L/hr for performing transesterification reaction.A mother liquid after the reaction was introduced to a buffer tank and was then continuously transported to distillation columns at a flow rate of 600 L/hr via a fifth delivery pump.The temperature of materials in a bottom of a first distillation column was controlled at between 140°C and 141°C, the temperature of a top of the first distillation column was controlled between 70 and 75.5°C, and a vacuum degree of -0.04 mPa.Ethanol was recovered at the top of the first distillation column, and a product produced in the bottom of the first distillation column was transported to a second distillation column.The temperature of materials in the second distillation column was controlled at between 90 and 95°C, the temperature of the top in the second distillation column was controlled at between 65 and 70°C, and the vacuum degree therein was controlled at -0.093 mPa. Heavy components were continuously obtained in the bottom of the second distillation column.Products produced in the top of the second distillation column were introduced into a third distillation column.The temperature in a bottom of the third distillation column was controlled at between 150 and 155°C, ethanol was continuously recovered from a top of the third distillation column, and 99.85 wt. % of ethyl lactate was continuously obtained at a flow rate of 500 L/hr from the bottom of the third distillation column.Lactoyl ethyl lactate and recovered ethanol were returned to the ethyl lactate synthesis system to participate the synthesis of ethyl lactate.

### Example 4

85 wt. % of L-lactic acid was purchased and used as a raw material.The raw material lactic acid was performed with vacuum distillation in a falling film evaporator.The temperature of the raw material in the falling film evaporator was maintained at 50°C, and a vacuum degree was maintained at 8000 Pa. The lactic acid after condensation contained 99 wt. % above of its monomer.500 kg of the lactic acid after condensation was transported to a polycondensation kettle via a first delivery pump, and a first catalyst was addedwith a weight ratio of the first catalyst to the lactic acid of 50/10000. The first catalyst was a mixture of zinc oxide, tin oxide, and butyl titanate at a ratio of 1:1:1.Materials were heated from 80°C to 120°C within 2 hours, and the vacuum degree was maintained at 5000 Pa. The whole reaction was performed in the stirring condition.The viscosity of a lactic acid oligomer was measured via an on line viscometer to determine whether a molecular weight thereof was between 1000 and 2500 according to corresponding relation between the viscosity and the molecular weight.If the molecular weight fell within the range, the lactic acid oligomer was transported to a depolymerization kettle by a height pressure difference and a vacuum pressure difference.A second catalyst was addedwith a weight ratio of the second catalyst to the lactic acid of 50/10000. The first catalyst was a mixture of zinc oxide, tin oxide, and butyl titanate at a ratio of 1:1:.The whole reaction was conducted in the stirring condition.Raw materials were rapidly heated to the temperature of 180°C, and the vacuum degree was controlled at 1000 Pa.Lactide vapor was continuously obtained, condensed, and accumulated in a collection tank.The depolymerization reaction stopped when the materials in the depolymerization kettle were completely depolymerized and the formation of the lactide became very slow.A liquid lactide in the collection tank was transported to a purification section via a second delivery pump where the liquid lactide was conducted with recrystallization purification process, and 385 kg of a refined lactide having a content of 99.7 wt. % above and an optical purity of 99.7% was obtained.The 385 kg of the refined L-lactide was transported to a dissolution kettle via a third delivery device while 500 kg of anhydrous methanol was added to the dissolution kettle. Materials therein were heated to the temperature of 75°C. After thematerials were completely dissolved, a fourth delivery pump operated to introduce the methanol solution comprising lactide to pass through a fixed bed reactor added with a composite catalyst for performing transesterification reaction.The composite catalyst is a mixture of a hydrogen-type cation exchange resin and a SO₄²⁻/TI₂O₄ solid high acidat a weight ratio of 1:2.A mother liquid after the reaction was introduced to a buffer tank and was then continuously transported to distillation columns via a fifth delivery pump.The temperature of materials in a bottom of a first distillation column was controlled at between 140°C and 141°C, the temperature of a top of the first distillation column was controlled between 65 and 65.5°C, and a pressure difference between the top and the bottom thereof was controlled at 1.8 kPa.Methanol was recovered at the top of the first distillation column, and a product produced in the bottom of the first distillation column was transported to a second distillation column.The temperature of materials in the second distillation column was controlled at between 90 and 95°C, the temperature of the top in the second distillation column was controlled at between 65 and 70°C, and the vacuum degree therein was controlled at -0.09 mPa. Heavy components were continuously obtained in the bottom of the second distillation column.Products produced in the top of the second distillation column were introduced into a third distillation column.The temperature in a bottom of the third distillation column was controlled at between 144 and 146°C, methanol was continuously recovered from a top of the third distillation column, and L-methyl lactate was continuously obtained from the bottom of the third distillation column.After the distillation, 600 kg of L-methyl lactate having a content of 99.85 wt. % and an optical purity of 99.9% was obtained.Lactoyl methyl lactate and recovered methanol were returned to the methyl lactate synthesis system to participate the synthesis of methyl lactate.

### Example 5

85 wt. % of D-lactic acid was purchased and used as a raw material.The raw material lactic acid was performed with vacuum distillation in a falling film evaporator.The temperature of the raw material in the falling film evaporator was maintained at 50°C, and a vacuum degree was maintained at 8000 Pa. The lactic acid after condensation contained 99 wt. % above of its monomer.500 kg of the lactic acid after condensation was transported to a polycondensation kettle via a first delivery pump, and a first catalyst was added with a weight ratio of the first catalyst to the lactic acid of 20/10000. The first catalyst was a mixture of zinc lactate, propionic acid, and butyl titanate at a weight ratio of 1:1:1.Materials were heated from 80°C to 120°C within 2 hours, and the vacuum degree was maintained at 5000 Pa.The whole reaction was performed in the stirring condition.The viscosity of a lactic acid oligomer was measured via an on line viscometer to determine whether a molecular weight thereof was between 1000 and 2500 according to corresponding relation between the viscosity and the molecular weight.

If the molecular weight fell within the range, the lactic acid oligomer was transported to a depolymerization kettle by a height pressure difference and a vacuum pressure difference.A second catalyst was addedwith a weight ratio of the second catalyst to the lactic acid of 50/10000. The second catalyst was a mixture of zinc lactate, propionic acid, and butyl titanate at a weight ratio of 1:1:1 The whole reaction was conducted in the stirring condition.Raw materials were rapidly heated to the temperature of 180°C, and the vacuum degree was controlled at 1000 Pa.Lactide vapor was continuously obtained, condensed, and accumulated in a collection tank.The depolymerization reaction stopped when the materials in the depolymerization kettle were completely depolymerized and the formation of the lactide became very slow.

A liquid lactide in the collection tank was transported to a purification section via a second delivery pump where the liquid lactide was conducted with recrystallization purification process, and 385 kg of a refined lactide having a content of 99.7 wt. % above and an optical purity of 99.7% was obtained.

The 385 kg of the refined L-lactide was transported to a dissolution kettle via a third delivery device while 500 kg of anhydrous methanol was added to the dissolution kettle.Materials therein were heated to the temperature of 75°C. After thematerials were completely dissolved, a fourth delivery pump operated to introduce the methanol solution comprising lactide to pass through a fixed bed reactor added with a composite catalyst for performing transesterification reaction.The composite catalyst is a mixture of a hydrogen-type cation exchange resin and a SO₄²⁻/TI₂O₄ solid high acid at a weight ratio of 1:2.A mother liquid after the reaction was introduced to a buffer tank and was then continuously transported to distillation columns via a fifth delivery pump. The temperature of materials in a bottom of a first distillation column was controlled at between 140°C and 141°C, the temperature of a top of the first distillation column was controlled between 65 and 65.5°C, and a pressure difference between the top and the bottom thereof was controlled at 1.8 kPa.Methanol was recovered at the top of the first distillation column, and a product produced in the bottom of the first distillation column was transported to a second distillation column.The temperature of materials in the second distillation column was controlled at between 90 and 95°C, the temperature of the top in the second distillation column was controlled at between 65 and 70°C, and the vacuum degree therein was controlled at -0.09 mPa. Heavy components were continuously obtained in the bottom of the second distillation column.Products produced in the top of the second distillation column were introduced into a third distillation column.The temperature in a bottom of the third distillation column was controlled at between 144 and 146°C, methanol was continuously recovered from a top of the third distillation column, and L-methyl lactate was continuously obtained from the bottom of the third distillation column.After the distillation, 600 kg of L-methyl lactate having a content of 99.85 wt. % and an optical purity of 99.9% was obtained.Lactoyl methyl lactate and recovered methanol were returned to the methyl lactate synthesis system to participate the synthesis of methyl lactate.

## Claims

1. A method for continuously producing a high-content high-optical-purity lactate, comprising the following steps:
A. polycondensation of lactic acid: adding a first catalyst to lactic acid, controlling a weight ratio of the first catalyst to lactic acid to be between 0.1/10000 and 100/10000, and conducting a polycondensation reaction, whereby yielding a lactic acid oligomer having a viscosity-average molecular weight of between 1000 and 2500;
B. depolymerization of an oligomer: adding a second catalyst to the lactic acid oligomer, controlling a weight ratio of the second catalyst to the lactic acid oligomer to be between 0.1/10000 and 100/10000, and conducting a depolymerization reaction, whereby yielding a crude lactide having a content of between 80 and 95 wt. %;
C. purification of lactide: refining the crude lactide to yield a refined lactide having a content of 99.5 wt. % above and an optical purity of 99.5 wt. % above and a purified mother liquid of an acyl lactate; and
D. synthesis of a high-optical-purity lactate: adding the refined lactide and an anhydrous organic alcohol at a weight ratio of between 1:0.7 and 1:5 to a dissolution kettle to completely dissolve lactide into the organic alcohol; introducing a resulting alcohol solution comprising lactide to pass through a fixed bed reactor added with a third catalyst so thattransesterification reaction between the lactide and the organic alcohol occurs, whereby yielding a lactate.

2. The method of claim 1, further comprising the following steps;
E. synthesis of electronic grade ethyl lactate: evenly mixing the purified mother liquid of the acyl lactate and anhydrous ethanol at a weight ratio of between 1:0.7 and 1:5 to yield a mixture, introducing the mixture to pass through a fixed bed reactor added with a fourth catalyst to obtain ethyl lactate; and
F. purification of the lactate: conducting purification and separation of the lactate.

3. The method of claim 1, further comprising: conducting vacuum distillation on lactic acid at a vacuum degree of between 10000 and 500 Pa and a distillation temperature of between 40 and 90°C before the polycondensation of lactic acid, and removing free water therefrom.

4. The method of claim 1, **characterized in that**
the first catalyst added in the polycondensation of lactic acid and the second catalyst added in the depolymerization of the oligomer are selected from the group consisting of a zinc catalyst, a tin catalyst, a titanium catalyst, an organic acid catalyst, and a mixture thereof;
a weight ratio of the first catalyst to the lactic acid is 50/10000; and
a weight ratio of the second catalyst to the lactic acid oligomer is 50/10000.

5. The method of claim 1 or 4, **characterized in that** the first catalyst added in the polycondensation of lactic acid and the second catalyst added in the depolymerization of the oligomer are selected from the group consisting of zinc lactate, zinc oxide, zinc dust, diethyl zinc, tin lactate, tin oxide, tin dioxide, stannous oxide, stannous lactate, stannous octoate, stannous chloride, tin powder, propionic acid, butyl titanate, and a composite catalyst thereof.

6. The method of claim 5, **characterized in that** the catalyst is a mixture of zinc oxide, tin oxide, and butyl titanate at a weight ratio of 1:1:1.

7. The method of claim 5, **characterized in that**the catalyst is a mixture of zinc lactate, propionic acid, and butyl titanate at a weight ratio of 1:1:1.

8. The method of claim 1, **characterized in that** the third catalyst added in the fixed bed reactor in the synthesis of the lactate is selected from the group consisting of a hydrogen-type cation exchange resin, a SO₄²⁻/TI₂O₄ solid high acid catalyst, and a composite catalyst thereof.

9. The method of claim 2, **characterized in that**
purification and separation of the lactate in step F) is performed using a tri-column distillation process comprising: removing excessive alcohol in a first distillation column,removing the acyl lactate in a second distillation column, and removing remaining alcohol in a third distillation column;
the first distillation column operates in decompression at a vacuum degree of between -0.02 and -0.08mPa, and an alcohol content accounts for between 1 and 5 wt. % of materials in a bottom of the first distillation column;
the second distillation column operates in decompression at a vacuum degree of between -0.086 and -0.098mPa, and a lactate content accounts for between 5 and 20 wt. % of materials in a bottom of the second distillation column;
the third distillation column operates at normal pressure, an alcohol content accounts for 0.1 wt. % of products in a bottom of the third distillation column; and
the lactate content reaches 99.8 wt. % after the tri-column distillation process.

10. The method of claim 9, **characterized in that** lactyl lactate overflowing from the bottom of the second distillation column is returned to a reactor kettle of step D) to participate the synthesis of the lactate.

11. The method of claim 9, **characterized in that** recovered alcohol obtained from tops of the first distillation column and the second distillation column is returned to the dissolution kettle in step D) to participate the dissolution of lactide.

12. The method of claim 1, **characterized in that**the raw material lactic acid is L-lactic acid or D-lactic acid.

13. The method of claim 1, **characterized in that**the anhydrous organic alcohol is methanol, ethanol, propanol, butanol, pentanol, hexanol, heptanol, octanol, nonanol, decanol, undecyl alcohol, dodecyl alcohol, tridecyl alcohol, hexadecanol, stearyl alcohol, lauryl alcohol, menthol, or glycerol alcohol.
